(19) Europäisches Patentamt
European Patent Office
Office européen des brevets

(11) Publication number : **0 522 887 A1**

(12) # EUROPEAN PATENT APPLICATION

(21) Application number : **92306403.4**

(22) Date of filing : **13.07.92**

(51) Int. Cl.[5] : **C07D 403/14,** A61K 31/415, C07D 403/12, C07D 401/14, A61K 31/40, A61K 31/44

(30) Priority : **12.07.91 GB 9115190**

(43) Date of publication of application : **13.01.93 Bulletin 93/02**

(84) Designated Contracting States : **PT**

(71) Applicant : **RHONE-POULENC RORER LIMITED**
**RPR House, St. Leonards Road**
**Eastbourne, East Sussex BN21 3YG (GB)**

(72) Inventor : **Pitchen, Philippe**
**c/o Rhone-Poulenc Rorer Limited**
**Dagenham, Essex RM10 7XS (GB)**
Inventor : **Smith, Christopher**
**c/o Rhone-Poulenc Rorer Limited**
**Dagenham, Essex RM10 7XS (GB)**
Inventor : **Thompson, David Michael**
**c/o Rhone-Poulenc Rorer Limited**
**Dagenham, Essex RM10 7XS (GB)**
Inventor : **Toft, Malcolm Percy**
**c/o Rhone-Poulenc Rorer Limited**
**Dagenham, Essex RM10 7XS (GB)**

(74) Representative : **Bentham, Stephen et al**
**J.A. Kemp & Co. 14 South Square Gray's Inn**
**London, WC1R 5LX (GB)**

(54) **Imidazoles as ACAT- and thromboxane TxA2 inhibitors.**

(57) Imidazole derivatives of the formula :-

$$[DPIM]\text{-}S(O)_p\text{-}W\text{-}Y \qquad I$$

wherein [DPIM] represents a 4,5-diphenylimidazol-2-yl grouping, p represents 0, 1 or 2, W represents a methylene group or an alkylene chain of 2 to 6 carbon atoms optionally substituted with one or more straight- or branched-chain alkyl groups of 1 to 4 carbon atoms, and Y represents a 5- or 6-membered unsaturated ring containing 1, 2, 3 or 4 nitrogen atoms, substituted by one or more 5- to 8-membered heterocyclic rings, each containing from 1 to 3 nitrogen atoms, the said 5- or 6-membered unsaturated ring being optionally further substituted by one or more straight- or branched-chain alkyl groups containing from 1 to 6 carbon atoms and the said 5- or 6-membered unsaturated ring being optionally further substituted by one or more substituents selected from straight- and branched-chain alkyl groups containing from 1 to 6 carbon atoms (which are each optionally substituted by a phenyl group), phenyl groups, amino groups and nitro groups, and pharmaceutically acceptable acid addition salts thereof possess useful pharmacological properties.

EP 0 522 887 A1

This invention relates to new therapeutically useful imidazole derivatives, to processes for their preparation and to pharmaceutical compositions containing them.

Thus, the present invention provides compounds of the general formula:-

$$[DPIM]\text{-}S(O)_p\text{-}W\text{-}Y \qquad I$$

wherein [DPIM] is as hereinafter depicted, wherein R and $R^1$ are the same or different and each represents a hydrogen or halogen (i.e. fluorine, chlorine, bromine or iodine) atom or a straight- or branched-chain alkyl or alkoxy group containing from 1 to about 6 carbon atoms, p represents 0, 1 or 2, W represents a methylene group or an alkylene chain of 2 to about 6 carbon atoms optionally substituted with one or more straight- or branched-chain alkyl groups of 1 to about 4 carbon atoms, and Y represents a 5- or 6-membered unsaturated ring containing 1, 2, 3 or 4 nitrogen atoms, substituted by one or more, preferably one, 5- to 8-membered heterocyclic rings, each containing from 1 to 3 nitrogen atoms, the said 5- or 6-membered unsaturated ring being preferably further substituted by one or more straight- or branched-chain alkyl groups containing from 1 to about 6 carbon atoms and the said 5- or 6-membered unsaturated ring being optionally further substituted by one or more substituents selected from straight- and branched-chain alkyl groups containing from 1 to about 6 carbon atoms (which are each optionally substituted by a phenyl group), phenyl groups, amino groups and nitro groups, and pharmaceutically acceptable acid addition salts thereof.

More particularly, Y represents a 5- or 6-membered unsaturated ring containing 1 or 2 nitrogen atoms, preferably a pyrazol-1-yl group, substituted by a 5- or 6-membered heterocyclic ring containing from 1 to 3, preferably 1, nitrogen atoms, e.g. a piperidyl, pyridyl or pyrrolyl group, the said 5- or 6-membered unsaturated ring being preferably further substituted by one or more straight- or branched-chain alkyl groups containing from 1 to about 6 carbon atoms, e.g. methyl or ethyl groups.

Compounds of particular importance include the following:-

A 2-[5-{5-methyl-3-(piperid-1-yl)pyrazol-1-yl}pentylthio]-4,5-diphenylimidazole
B 2-[5-{5-methyl-3-(4-pyridyl)-1-pyrazolyl}pentylthio]-4,5-diphenylimidazole
C 2-[5-{5-methyl-3-(3-pyridyl)-1-pyrazolyl}pentylthio]-4,5-diphenylimidazole
D 2-[5-{5-methyl-3-(2-pyridyl)-1-pyrazolyl}pentylthio]-4,5-diphenylimidazole
E 2-[5-{5-methyl-3-(1-pyrrolyl)pyrazol-1-yl}pentylthio]-4,5-diphenylimidazole
F (±)-2-[5-{5-methyl-3-(1-piperidyl)-1-pyrazolyl}pentylsulphinyl]-4,5-diphenylimidazole
G 2-[5-{5-methyl-3-(1-piperidyl)-1-pyrazolyl}pentylsulphonyl]-4,5-diphenylimidazole and
H 3-(4-pyridyl)-5-methyl-1-[5-{4,5-diphenylimidazol-2-yl)sulphinyl}pentyl]pyrazole.

The letters A to H are allocated to the compounds for easy reference later in this specification.

Compounds within the scope of the present invention exhibit positive pharmacological activities as demonstrated by the following tests which are believed to correlate to pharmacological activity in humans and other animals.

The said compounds have valuable pharmacological properties, in particular properties which are indicative of utility in the treatment of conditions which can be ameliorated by the administration of an inhibitor of acyl coenzyme-A:cholesterol-O-acyl transferase (ACAT; EC 2.3.1.26), such as atherosclerosis, hyperlipidaemia, cholesterol ester storage disease and atheroma in vein grafts, and reduction of atherosclerotic plaque, or of an inhibitor of the binding of thromboxane $TxA_2$ to its receptors, such as thrombosis and myocardial infarction, vasospastic disorders, for example associated with angina, and bronchospasm, for example associated with asthma, or in reperfusion salvage therapy, for example after ischaemic injury.

The present invention also provides a method for the treatment of a human or animal patient suffering from, or subject to, conditions which can be ameliorated by the administration of an inhibitor of acyl coenzyme-A:cholesterol-O-acyl transferase, such as atherosclerosis, hyperlipidaemia, cholesterol ester storage disease and atheroma in vein grafts, and reduction of atherosclerotic plaque, or of an inhibitor of the binding of thromboxane $TxA_2$ to its receptors, such as thrombosis and myocardial infarction, vasospastic disorders, for example associated with angina, and bronchospasm, for example associated with asthma, or in reperfusion salvage therapy, for example after ischaemic injury, which comprises administering to the patient an effective amount of a compound of formula (I), or a pharmaceutically acceptable acid addition salt thereof, as hereinbefore defined, to secure an improvement in the condition of the patient.

The invention also provides a compound of formula (I), or a pharmaceutically acceptable acid addition salt thereof, as hereinbefore defined, for use in a new method of treatment, of the human or animal body, by therapy, of conditions which can be ameliorated by the administration of an inhibitor of acyl coenzyme-A:cholesterol-O-acyl transferase, such as atherosclerosis, hyperlipidaemia, cholesterol ester storage disease and atheroma in vein grafts, and reduction of atherosclerotic plaque, or of an inhibitor of the binding of thromboxane $TxA_2$ to its receptors, such as thrombosis and myocardial infarction, vasospastic disorders, for example associated with angina, and bronchospasm, for example associated with asthma, or in reperfusion salvage therapy, for example after ischaemic injury.

The present invention also provides pharmaceutical formulations which contain at least one of the compounds of formula (I), or a pharmaceutically acceptable salt thereof, as hereinbefore defined, in association with a pharmaceutically acceptable carrier or coating.

In assays performed in-vitro, microsomes, obtained from the tissues of rabbits fed on a diet supplemented with cholesterol for 7 days, were incubated with radiolabelled oleoyl-CoA in the presence of compounds according to the invention at various concentrations. The degree of ACAT inhibition was measured and the $IC_{50}$ was calculated, and found to be of the order of 3nM.

Compounds of general formula (I) can be prepared by the application or adaptation of known methods, by which is meant methods used heretofore or described in the literature.

According to a feature of the invention, compounds of general formula (I), as hereinbefore defined, are prepared by the reaction of a compound of the general formula:-

[DPIM]-SH (II)

or a salt thereof, wherein [DPIM] is as hereinbefore defined, with a compound of formula:

X-W-Y (III)

or a salt thereof, wherein X is a group displaceable by a thiolate salt, such as a halogen e.g. a chlorine, bromine or iodine, atom or an alkyl- or arylsulphonyloxy group (e.g. methanesulphonyloxy or 4-toluenesulphonyloxy) and W and Y are as hereinbefore defined. The reaction is generally carried out in an inert organic solvent such as tetrahydrofuran or dimethylformamide at a temperature from ambient to 110°C and optionally in the presence of a proton acceptor, such as an amine (e.g. triethylamine).

According to a further feature of the present invention, compounds of general formula (I) wherein Y represents a 5- or 6-membered unsaturated ring containing 1, 2, 3 or 4 nitrogen atoms, substituted by a pyrrol-1-yl group are prepared from the corresponding compound wherein the said 5- or 6-membered unsaturated ring is instead substituted by an amino group, by reaction with a 2,5-dialkoxytetrahydrofuran, preferably in acidic conditions and at an elevated temperature.

According to a further feature of the present invention, compounds of general formula (I) are prepared by the interconversion of other compounds of general formula (I). For example, compounds of formula (I), wherein [DPIM], R, $R^1$, W and Y are as hereinbefore defined and p represents 1 or 2, are prepared by the oxidation of compounds of formula (I), wherein [DPIM], R, $R^1$, W and Y are as hereinbefore defined and p is less than in the desired product.

The oxidation may be performed by using a conventional oxidant, such as hydrogen peroxide, sodium metaperiodate, a hypochlorite, an acyl nitrite, a peroxomonosulphate such as tetrabutylammonium peroxomonosulphate, sodium perborate, peracids, such as percarboxylic acids (e.g. m-chloroperbenzoic acid), potassium permanganate or potassium hydrogen persulphate, or a ruthenium (VIII) compound, in an inert solvent, at or below room temperature. Ruthenium (VIII) compounds can conveniently be prepared in situ by the oxidation of ruthenium (III) compounds, for example by means of a percarboxylic acid (e.g. m-chloroperbenzoic acid).

Suitable solvents may include water, alcohols, water-alcohol mixtures, chlorinated hydrocarbons, such as dichloromethane, and organic acids.

Compounds of general formula (I) wherein p is 1, the other symbols being as hereinbefore defined, may be obtained in a chirally pure form by separation of the enantiomers arising from a non-selective oxidation or by using known enantio-selective oxidising systems.

For example, according to a further feature of the present invention, one enantiomer of such a sulphoxide of formula I, free or virtually free from the other enantiomer, is prepared from the appropriate enantiomer of a compound of the general formula IV, hereinafter depicted, wherein R, $R^1$, W and Y are as hereinbefore defined, and Z represents a protective group, for example a trialkylsilylalkoxymethyl group, e.g. a trialkylsilylethoxymethyl group, preferably a trimethylsilylethoxymethyl group, by the replacement of the said protective group by a hydrogen atom. The reaction can be carried out by hydrolysis, for example by using a dilute acid, e.g. hydrochloric acid, or alternatively by reaction with tetrabutylammonium fluoride, preferably in an ethereal medium, e.g. tetrahydrofuran.

The appropriate enantiomer of the compound of the general formula IV, hereinbefore defined, free or virtually free from the undesired enantiomer, can be prepared by the reaction of the appropriate enantiomer of a compound of general formula V, hereinafter depicted, wherein R, $R^1$, W and Z are as hereinbefore defined, and $X^2$ represents a halogen, preferably chlorine, atom, with a compound of formula Y-H, or a salt thereof, by the application or adaptation of conditions similar to those hereinbefore described for the reaction of compounds of formula II with compounds with compounds of formula III.

The appropriate enantiomer of the compound of the general formula V, wherein R, $R^1$, W, $X^2$ and Z are as hereinbefore defined, free or virtually free from the undesired enantiomer, can be prepared from the appropriate enantiomer of a compound of general formula VI hereinafter depicted, wherein R, $R^1$ and Z are as hereinbefore defined, by the application or adaptation of known methods, for example, When W is other than methylene, by

reaction with a compound of the general formula:-

$$X^3\text{-}W'\text{-}X^2 \qquad \text{VII}$$

wherein $X^2$ is as hereinbefore defined, $X^3$ represents a halogen, preferably iodine, atom, and W′ represents a methylene group or an alkylene chain of 2 to about 5 carbon atoms optionally substituted with one or more straight or branched alkyl groups of 1 to about 4 carbon atoms, or a salt thereof, by the application or adaptation of conditions similar to those hereinbefore described for the reaction of compounds of formula II With compounds with compounds of formula III.

According to a further feature of the present invention, the appropriate enantiomer of the compound of the general formula VI, hereinbefore defined, free or virtually free from the undesired enantiomer, is prepared by the enantio-selective oxidation of a compound of general formula VIII, hereinafter depicted, Wherein R, $R^1$ and Z are as hereinbefore defined.

The enantio-selective oxidation may be carried out by the application or adaptation of known enantio-selective oxidising systems, for example a mixture of diethyl D-tartrate or diethyl L-tartrate with titanium isopropoxide and an organic hydroperoxide, e.g. tert-butyl hydroperoxide or, preferably, cumene hydroperoxide, in a suitable solvent such as dichloromethane, preferably under an inert atmosphere.

According to a further feature of the present invention, one enantiomer of a compound of general formula (I) wherein p is 1, the other symbols being as hereinbefore defined, may be obtained, free or virtually free from the other enantiomer, by reaction of a corresponding compound of general formula (I) wherein p is 0, the other symbols being as hereinbefore defined, with a known enantio-selective oxidising system. A particularly effective example of such a system involves the use of a chiral oxaziridine oxidising agent. Such agents are well known in the art, and particularly suitable agents are the so-called "(+)-8,8-dichlorocamphorylsulfonyloxaziridine", whose structure is depicted hereinafter, and its enantiomer, which are items of commerce. Their preparation and use are described, for example, by Davis et al., J. Org. Chem., (1990), 55, 3715-3717.

By the term "pharmaceutically acceptable salts" as used in this specification is meant salts the anions of which are relatively innocuous to the animal organism when used in therapeutic doses so that the beneficial pharmaceutical properties of the parent compounds of general formula (I) are not vitiated by side-effects ascribable to those anions.

Suitable acid addition salts for use in pharmaceuticals may be selected from salts derived from inorganic acids, for example hydrochlorides, hydrobromides, phosphates, sulphates and nitrates, and organic acids, for example oxalates, lactates, tartrates, acetates, salicylates, citrates, propionates, succinates, fumarates, maleates, methylene-bis-β-hydroxynaphthoates, gentisates and di-p-toluoyltartrates.

According to a further feature of the invention, salts of compounds of formula (I) are prepared by reaction of the parent compounds of formula (I) with the appropriate acid, by the application or adaptation of known methods.

As well as being useful in themselves as active compounds, salts of compounds of formula (I) are useful for the purposes of purification of the parent compounds of formula (I), for example by exploitation of the solubility differences between the salts and the parent compounds, by techniques well known to those skilled in the art.

The parent compounds of formula (I) can be regenerated from their salts by the application or adaptation of known methods.

For example, parent compounds of general formula (I) can be regenerated from their acid addition salts by treatment with an alkali, e.g. aqueous sodium bicarbonate solution or aqueous ammonia solution.

In this specification reference to compounds of formula (I) is intended to include reference to their pharmaceutically acceptable salts, where the context so permits.

The starting materials and intermediates can be prepared by the application or adaptation of known methods.

Compounds of formula (I) can be purified by the usual physical means, for example by crystallisation or chromatography.

The following Examples illustrate the preparation of the compounds according to the invention.

Nuclear magnetic resonance (NMR) spectra were recorded at 200MHz or 400MHz. Chemical shifts are expressed in ppm relative to tetramethylsilane.

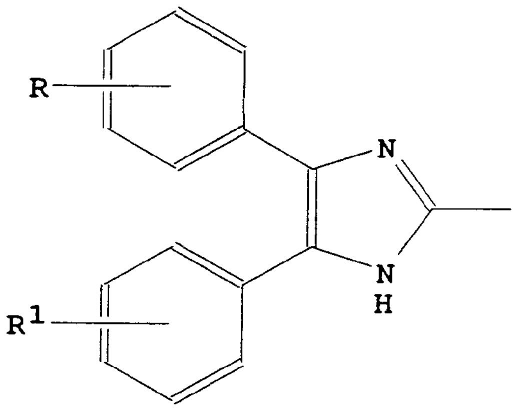

[DPIM]

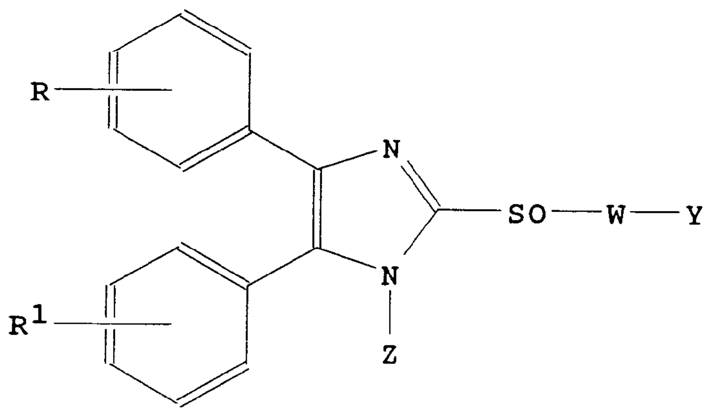

IV

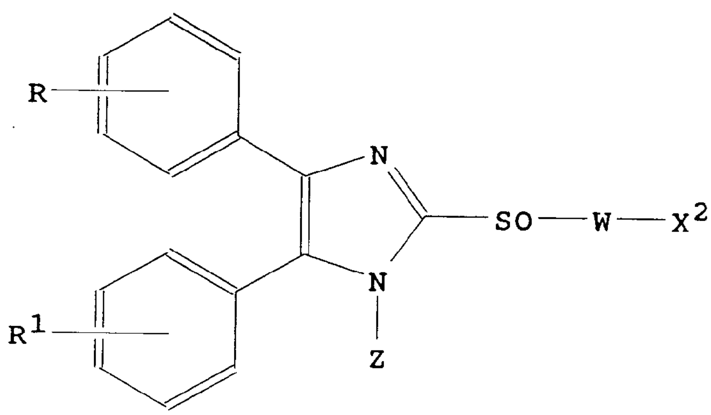

V

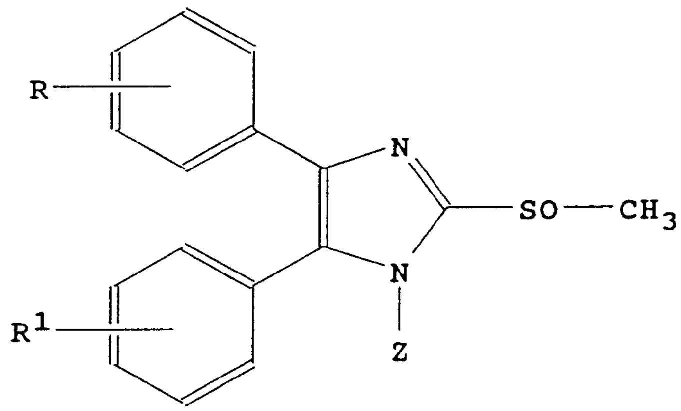

VI

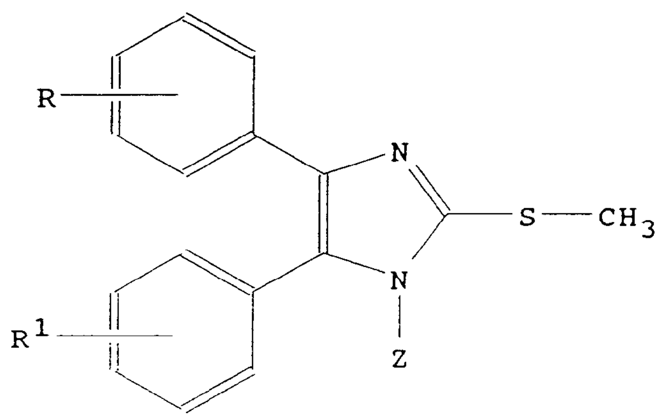

VIII

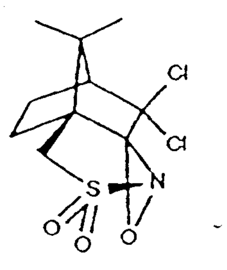

"(+)-8,8-dichlorocamphorylsulfonyloxaziridine"

EXAMPLE 1

Compounds A, B, C and D

A stirred solution of 3-amino-5-methylpyrazole (25g) in anhydrous tetrahydrofuran (500ml) under nitrogen was carefully treated with sodium hydride (60% dispersion in oil, 8.7g). After stirring for 30 minutes at room temperature 1-bromo-5-chloropentane (49g) was added and the mixture was refluxed for 48 hours, allowed to cool to room temperature, and filtered to remove sodium chloride which was washed with tetrahydrofuran

(50ml). The combined filtrate and washings were evaporated and the resulting light brown liquid was dissolved in tetrahydrofuran (100ml). This solution was added to a stirred suspension of the sodium salt of 4,5-diphenylimidazole-2-thiol in anhydrous tetrahydrofuran [prepared by carefully treating a suspension of 4,5-diphenylimidazole-2-thiol (64.84g) in anhydrous tetrahydrofuran (500ml), under nitrogen, with sodium hydride (60% dispersion in oil, 9.4g) and stirring at room temperature for 40 minutes] and the whole was heated under reflux for 48 hours. The mixture was evaporated, treated with aqueous hydrochloric acid (1500ml,2N) and charcoal (4g) at 90°C and then it was filtered. The cooled filtrate was basified by addition of solid potassium carbonate and extracted five times with dichloromethane (500ml). The combined dichloromethane extracts were dried over magnesium sulphate, evaporated and the residue (40g) was purified by flash chromatography (using as eluent a mixture of dichloromethane and methanol, 95:5v/v). The major component was crystallised from a mixture of ethyl acetate and diethyl ether (1:1v/v), to give 2-[5-{5-methyl-3-(piperid-1-yl)pyrazol-1-yl}pentylthio]-4,5-diphenylimidazole (8g) as a white solid, m.p. 156-158°C. [Found:- C,71.2;H,7.2;N,14.0;S,6.82%; Calculated:- C,71.7;H,7.26;N,14.4;S,6.6%].

Also isolated was 2-[5-(3-amino-5-methylpyrazol-1-yl)pentylthio]-4,5-diphenylimidazole, m.p.60-62°C.

By proceeding in a similar manner but replacing the 3-amino-5-methylpyrazole by the appropriate pyrazoles and purifying the appropriately substituted intermediate 1-(5-chloropentyl)pyrazole derivative, there were prepared:-

2-[5-{5-methyl-3-(4-pyridyl)-1-pyrazolyl}pentylthio]-4,5-diphenylimidazole ,m.p. 182-184°C [Found:- C, 72.4; H, 6.14; N, 14.6; S, 6.57%; Calculated:- C, 72.6; H, 6.09; N, 14.6; S, 6.68%];

2-[5-{5-methyl-3-(3-pyridyl)-1-pyrazolyl}pentylthio]-4,5-diphenylimidazole, m.p. 65-67°C [Found:- C, 72.7; H, 6.15; N, 14.5; S, 6.63%; Calculated:- C, 72.6; H, 6.09; N, 14.6; S, 6.68%]; and

2-[5-{5-methyl-3-(2-pyridyl)-1-pyrazolyl}pentylthio]-4,5-diphenylimidazole, m.p. 50-52°C
[Found:- C, 73.1; H, 6.18; N, 14.6; S, 6.59%;
Calculated:- C, 72.6; H, 6.09; N, 14.6; S, 6.68%].

## EXAMPLE 2

### Compound E

2,5-Dimethoxytetrahydrofuran (0.86g) was added to a solution of 2-[5-(3-amino-5-methylpyrazol-1-yl)pentylthio]-4,5-diphenylimidazole (2g) in acetic acid (50ml) and the reaction mixture was heated at reflux for 40 minutes. After cooling to room temperature the reaction mixture was evaporated and the residual dark brown oil purified by flash chromatography (eluting with a mixture of dichloromethane and methanol, 95:5v/v) giving as the major component 2-[5-{5-methyl-3-(1-pyrrolyl)pyrazol-1-yl}pentylthio]-4,5-diphenylimidazole (0.3g) as an amorphous white solid from diethyl ether, m.p.147-149°C [Found:- C, 71.9; H, 6.26; N, 14.8; S, 6.82%; Calculated:- C, 71.9; H, 6.25; N, 15.0; S, 6.85%].

## EXAMPLE 3

### Compound F

A stirred solution of 2-[5-{5-methyl-3-(piperid-1-yl)pyrazol-1-yl}pentylthio]-4,5-diphenylimidazole (2g) in dichloromethane (40ml) cooled to below -10°C and meta-chloroperbenzoic acid (1.18g of 50-60%) was added portionwise over 15 minutes maintaining the reaction temperature below -10°C. After stirring for 20 minutes at -10°C a further quantity of meta-chloroperbenzoic acid (0.6g) was added over 5 minutes whilst maintaining the reaction temperature at -5 to -10°C. Stirring was continued for a further 10 minutes by which time tlc indicated almost complete reaction. An 10% aqueous solution of sodium metabisulphite was added and the organic phase was separated, washed with 10% aqueous sodium bicarbonate solution (2 x 50ml) then dried over magnesium sulphate. Evaporation, followed by flash chromatography (eluting with ethyl acetate) gave as the major component (±)-2-[5-{5-methyl-3-(1-piperidyl)-1-pyrazolyl}pentylsulphinyl]-4,5-diphenylimidazole (a hydrate) (0.5g), as a pale yellow amorphous solid.

## EXAMPLE 4

### Compound F

A solution of 2-[5-{5-methyl-3-(1-piperidyl)-1-pyrazolyl}pentylthio]-4,5-diphenylimidazole (5g) in anhydrous dichloromethane was treated, in one portion, with tetrabutylammonium peroxomonosulphate (10g). The

clear solution was left at ambient temperature for 18 hours, evaporated and the residue treated with ethyl acetate (100ml) and water (100ml). The organic phase was separated, washed with water (4 x 100ml), dried over magnesium sulphate and evaporated. The residual oil (2.6g) was purified by flash chromatography (eluting with a mixture of dichloromethane and methanol, 98:2v/v) and the major component was crystallised from ethyl acetate to give (±)-2-[5-{5-methyl-3-(1-piperidyl)-1-pyrazolyl}pentylsulphinyl]-4,5-diphenylimidazole (1.1g) as off-white microcrystals, m.p. 153-155°C [Found:- C, 69.0; H, 7.10; N, 13.5%; Calculated:- C, 69.4; H, 7.03; N, 14.0%].

EXAMPLE 5

Compound G

2-[5-{5-methyl-3-(1-piperidyl)-1-pyrazolyl}pentylthio]-4,5-diphenylimidazole (6g) was added with stirring to trifluoracetic acid (120ml). After the initially formed sticky suspension had dissolved (about 10 minutes) sodium perborate tetrahydrate (4.8g) was added in one portion. The solid slowly dissolved to give a yellow solution which became successively green, blue, and finally deep violet over a period of 30 minutes. After stirring at room temperature for 2 hours ferrous sulphate heptahydrate (18g) was added and stirring was continued for a further 10 minutes. The reaction mixture was poured into water (900ml) and extracted twice with dichloromethane (450ml). The combined extracts were washed with saturated aqueous sodium bicarbonate solution, dried over magnesium sulphate and evaporated. The residual solid (6.1g) was subjected to flash chromatography (eluting with a mixture of dichloromethane and methanol, 95:5v/v), and the major product was crystallised from ethyl acetate to give 2-[5-{5-methyl-3-(1-piperidyl)-1-pyrazolyl}pentylsulphonyl]-4,5-diphenylimidazole (1.1g), as off-white crystals, m.p.159-161°C; [Found:- C, 67.1; H, 6.94; N, 13.2%; Calculated:- C, 67.3; H, 6.82; N, 13.5%].

EXAMPLE 6

A solution of diethyl D-tartrate (24.72g) in dry dichloromethane (430ml) stirred at room temperature under nitrogen was treated successively with titanium isopropoxide (17.04g) and distilled water (1ml). After 15 minutes stirring, a solution of 2-methylthio-4,5-diphenyl-1-[(2-trimethylsilyl)ethoxymethyl]imidazole (23.76g) in dry dichloromethane (50ml) was added and the mixture was cooled to -20°C. It was then treated with cumene hydroperoxide (22.8g; 80%) at -20°C during 10 minutes and the solution was stirred under nitrogen for 24 hours at -18 to -20C. It was then treated with distilled water (22ml) and the mixture was vigorously stirred for 1 hour at room temperature. The gel obtained was filtered and washed with dichloromethane (4x300ml). The combined filtrate was treated with dilute aqueous sodium hydroxide solution (500ml;2N), stirred for 1 hour, washed with brine (500ml), dried over magnesium sulphate, and evaporated to dryness. The residue was subjected to flash chromatography on silica gel, eluting with a mixture of cyclohexane and ethyl acetate (7:3v/v), to give an oil, which was crystallised from pentane to give an enantiomer of 2-methylsulphinyl-4,5-diphenyl-1-[2-(trimethylsilyl)ethoxymethyl]imidazole (16.8g) in the form of a white solid, m.p. 67-70°C. [Enantiomeric excess: 91%, determined by chiral HPLC (column: Chiralcel OD; mobile phase: isopropanol:heptane 4:96; UV detection at 270nm) $[\alpha]_D^{22}$ = +3.7° (c 1.09; dichloromethane) NMR ($CDCl_3$):- 0.00 (s,9H); 0.90 (t, 2H); 3.30 (s, 3H); 3.52 (dd,2H); 5.47 (dd, 2H); 7.20-7.22 (m, 10H)].

EXAMPLE 7

To a solution of 16g of 2-methylsulphinyl-4,5-diphenyl-1-[2-(trimethylsilyl)ethoxymethyl]imidazole (as obtained in Example 6) in 200ml of dry tetrahydrofuran stirred at -70°C under nitrogen were successively added 20ml hexamethylphosphoramide and, during 10 minutes, 21.32ml of a 2M solution of lithium diisopropylamide in heptane and tetrahydrofuran. The temperature was allowed to warm up to -25°C and a solution of 11g of 5-chloro-1-iodobutane in 30ml dry tetrahydrofuran was added over 3 minutes at -20 to -25°C. The yellow solution was stirred 15 minutes at -20°C and warmed up to room temperature during 1 hour. 100ml of a 5% aqueous solution of ammonium chloride were added to the solution and the mixture was extracted with diethyl ether (3x150ml). The combined extracts were washed with 100ml of 2N hydrochloric acid (twice), 100ml water, 100ml brine, dried over magnesium sulphate and evaporated to dryness. The residue was subjected to flash chromatography on silica gel, eluting with a mixture of cyclohexane and ethyl acetate (75:25), to give an enantiomer of 2-(5-chloropentylsulphinyl)-4,5-diphenyl-1-[2-(trimethylsilyl)ethoxymethyl]imidazole (17g), in the form of an oil. [NMR ($CDCl_3$):- 0.00 (s,9H); 0.90 (dd,2H); 1.65-2.05 (m,6H); 3.40-3.73 (dm,2H); 3.49 (dd,2H); 3.59 (t,2H); 5.46 (dd,2H); 7.19-7.55 (m,10H)].

EXAMPLE 8

To a solution of sodium hydride (0.43g of a 60% dispersion in mineral oil) in 50ml of dry dimethylformamide stirred under nitrogen was added a solution of 2g of 3-(4-pyridyl)-5-methylpyrazole in 20ml of dry dimethylformamide over 10 minutes at a temperature maintained below 30°C. After stirring at room temperature for 30 minutes, 1.59g of sodium iodide was added. A solution of 4.1g of 2-(5-chloropentylsulphinyl)-4,5-diphenyl-1-[2-(trimethylsilyl)ethoxymethyl]imidazole (as obtained from Example 7) in 20ml of dry dimethylformamide was then added over 1 minute and the solution stirred at 70°C for 1 hour. The mixture was cooled to room temperature and poured into 600ml of water. The mixture was extracted 3 times with 100ml diethyl ether. The combined extracts were washed with 200ml water (twice), 200ml brine, dried over magnesium sulphate and evaporated to dryness to give 4.93g of an enantiomer of 3-(4-pyridyl)5-methyl-1-[5-{4,5-diphenyl-1-(2-trimethylsilylethoxymethyl)imidazol-2-yl)sulphinyl}pentyl]pyrazole in the form of a yellow oil which was used in Example 9 without further purification.

[NMR ($CDCl_3$):- 0.00 (s,9H); 0.89 (dd,2H); 1.55 to 2.08 (m,6H); 2.37 (s,3H); 3.40 to 3.75 (m,4H); 4.16 (t,2H); 5.46 (dd,2H); 6.46 (s,1H); 7.20 to 7.55 (m,10H); 7.72 (dd,2H); 8.62 (dd,2H)].

EXAMPLE 9

Compound H

37ml of 1N hydrochloric acid were added to a solution of 2.3g of crude 3-(4-pyridyl)-5-methyl-1-[5-{4,5-diphenyl-1-(2-trimethylsilylethoxymethyl)imidazol-2-yl)sulphinyl}pentyl]pyrazole (as obtained in Example 8) in 37ml of ethanol and the mixture stirred at 55°C for 1 hour. The solution was then adjusted to pH8 at room temperature with IN sodium hydroxide and extracted with 100ml of ethyl acetate (twice). The combined extracts were washed with 100ml of water (twice), 100ml brine, dried over magnesium sulphate and evaporated to dryness. The residue was subjected to flash chromatography (on 40g silica gel, eluting with a mixture of methanol and ethyl acetate, 5:95v/v), to give an oil, which was crystallised by trituration in 5ml of ethyl acetate to give 0.791g of an enantiomer of 3-(4-pyridyl)-5-methyl-1-[5-{4,5-diphenylimidazol-2-yl)sulphinyl}pentyl]pyrazole in the form of a white crystalline solid; m.p = 172-3°C.

[Enantiomeric excess: 97%, determined by chiral HPLC (column: Chiralcel OD; mobile phase heptane:isopropanol: methanol 500:20:20; UV detection at 270nm).

$[\alpha]_D^{22}$ = +18.6° (c 0.97;dichloromethane)

[NMR ($CDCl_3$):- 1.40 to 2.15 (m,7H); 2.29 (s,3H); 3.17 to 3.31 (m,2H); 4.05 (t,2H); 6.40 (s,1H); 7.25 to 7.54 (m,10H); 7.66 (d,2H); 8.56 (d,2H)].

The present invention also includes within its scope pharmaceutical formulations which comprise at least one of the compounds of formula (I) or a pharmaceutically acceptable salt thereof in association with a pharmaceutically acceptable carrier or coating. In clinical practice the compounds of the present invention may be administered parenterally, rectally or orally.

Solid compositions for oral administration include compressed tablets, pills, powders and granules. In such solid compositions, one or more of the active compounds is, or are, admixed with at least one inert diluent such as starch, sucrose or lactose. The compositions may also comprise, as is normal practice, additional substances other than inert diluents, e.g. lubricating agents, such as magnesium stearate.

Liquid compositions for oral administration include pharmaceutically acceptable emulsions, solutions, suspensions, syrups and elixirs containing inert diluents commonly used in the art such as water and liquid paraffin. Besides inert diluents such compositions may comprise adjuvants, such as wetting and suspending agents, and sweetening, flavouring, perfuming and preserving agents. The compositions according to the invention for oral administration also include capsules of absorbable material such as gelatin, containing one or more of the active substances with or without the addition of diluents or excipients.

Compositions according to the invention for parenteral administration include sterile aqueous, aqueous-organic, and organic solutions, suspensions and emulsions. Examples of organic solvents or suspending media are propylene glycol, polyethylene glycol, vegetable oils such as olive oil and injectable organic esters such as ethyl oleate. The compositions may also contain adjuvants such as stabilising, preserving, wetting, emulsifying and dispersing agents. They may be sterilised, for example, by filtration through a bacteria-retaining filter, by incorporation in the compositions of sterilising agents, by irradiation or by heating. They may also be manufactured in the form of sterile solid compositions, which can be dissolved in sterile water or some other sterile injectable medium immediately before use.

Solid compositions for rectal administration include suppositories formulated in accordance with known methods and containing at least one compound of formula (I) or a pharmaceutically acceptable salt thereof.

The percentage of active ingredient in the compositions of the invention may be varied, it being necessary that it should constitute a proportion such that a suitable dosage shall be obtained. Obviously, several unit dosage forms may be administered at about the same time. The dose employed will be determined by the physician, and depends upon the desired therapeutic effect, the route of administration, the duration of the treatment and the condition of the patient. In the adult, the doses are generally from about 0.01 to about 100, preferably about 0.1 to about 70, more especially about 0.5 to about 10, mg/kg body weight per day by oral administration, and from about 0.001 to about 10, preferably about 0.01 to about 1, mg/kg body weight per day by intravenous administration.

The following Examples illustrate pharmaceutical compositions according to the present invention.

COMPOSITION EXAMPLE 1

No. 2 size gelatin capsules each containing:-

| | |
|---|---|
| 3-(4-pyridyl)-5-methyl-1-[{2-(4,5-diphenyl-imidazolyl)}sulphinylpentyl]pyrazole | 20 mg |
| lactose | 100 mg |
| starch | 60 mg |
| dextrin | 40 mg |
| magnesium stearate | 1 mg |

were prepared in accordance with the usual procedure.

COMPOSITION EXAMPLE 2

No. 2 size gelatin capsules each containing:-

| | |
|---|---|
| 2-[5-{5-methyl-3-(piperid-1-yl)pyrazolyl}pentylthio]-4,5-diphenylimidazole | 20 mg |
| lactose | 100 mg |
| starch | 60 mg |
| dextrin | 40 mg |
| magnesium stearate | 1 mg |

were prepared in accordance with the usual procedure.

## Claims

**1.** An imidazole derivative of the general formula:-

$$[DPIM]-S(O)_p-W-Y \qquad I$$

wherein [DPIM] represents a grouping of the formula:

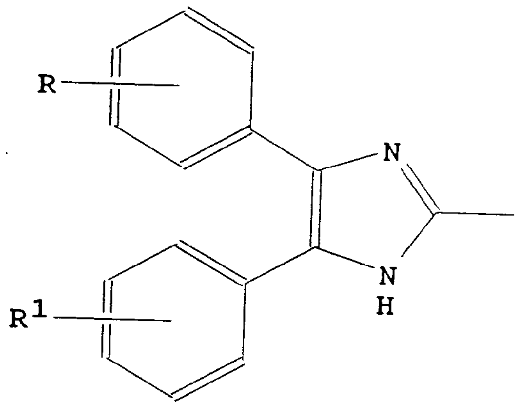

[DPIM]

wherein R and $R^1$ are the same or different and each represents a hydrogen or halogen atom or a straight- or branched-chain alkyl or alkoxy group containing from 1 to 6 carbon atoms, p represents 0, 1 or 2, W represents a methylene group or an alkylene chain of 2 to 6 carbon atoms optionally substituted with one or more straight- or branched-chain alkyl groups of 1 to 4 carbon atoms, and Y represents a 5- or 6-membered unsaturated ring containing 1, 2, 3 or 4 nitrogen atoms, substituted by one or more 5- to 8-membered heterocyclic rings, each containing from 1 to 3 nitrogen atoms, the said 5- or 6-membered unsaturated ring being optionally further substituted by one or more straight- or branched-chain alkyl groups containing from 1 to 6 carbon atoms and the said 5- or 6- membered unsaturated ring being optionally further substituted by one or more substituents selected from straight- and branched-chain alkyl groups containing from 1 to 6 carbon atoms (which are each optionally substituted by a phenyl group), phenyl groups, amino groups and nitro groups, or a pharmaceutically acceptable acid addition salt thereof.

2. A compound according to claim 1 wherein Y represents a 5- or 6-membered unsaturated ring containing 1 or 2 nitrogen atoms substituted by a 5- or 6-membered heterocyclic ring containing from 1 to 3 nitrogen atoms.

3. A compound according to claim 1 or 2 wherein the 5- or 6-membered unsaturated ring is further substituted by one or more straight- or branched-chain alkyl groups containing from 1 to 6 carbon atoms.

4. A compound according to claim 1, 2 or 3 wherein Y represents a pyrazol-1-yl group.

5. A compound according to claim 2, 3 or 4 wherein the 5- or 6-membered heterocyclic ring contains 1 nitrogen atom.

6. A compound according to claim 2, 3 or 4 wherein the 5- or 6-membered heterocyclic ring is piperidyl, pyridyl or pyrrolyl.

7. A compound according to any one of the preceding claims wherein the 5- or 6-membered unsaturated ring is further substituted by one or more methyl or ethyl groups.

8. A compound according to claim 1 which is
   2-[5-{5-methyl-3-(piperid-1-yl)pyrazol-1-yl}pentylthio]-4,5-diphenylimidazole,
   2-[5-{5-methyl-3-(4-pyridyl)-1-pyrazolyl}pentylthio]-4,5-diphenylimidazole,
   2-[5-{5-methyl-3-(3-pyridyl)-1-pyrazolyl}pentylthio]-4,5-diphenylimidazole,
   2-[5-{5-methyl-3-(2-pyridyl)-1-pyrazolyl}pentylthio]-4,5-diphenylimidazole,
   2-[5-{5-methyl-3-(1-pyrrolyl)pyrazol-1-yl}pentylthio]-4,5-diphenylimidazole,
   (±)-2-[5-{5-methyl-3-(1-piperidyl)-1-pyrazolyl}pentylsulphinyl]-4,5-diphenylimidazole,
   2-[5-{5-methyl-3-(1-piperidyl)-1-pyrazolyl}pentylsulphonyl]-4,5-diphenylimidazole, or
   3-(4-pyridyl)-5-methyl-1-[5-{4,5-diphenylimidazol-2-yl)sulphinyl}pentyl]pyrazole,
   or a pharmaceutically acceptable acid addition salt thereof.

9. A process for the preparation of a compound of general formula (I), as defined in claim 1 which comprises:
   (A) the reaction of a compound of the general formula:-

[DPIM]-SH (II)

or a salt thereof, wherein [DPIM] is as defined in claim 1, with a compound of formula:

X-W-Y (III)

or a salt thereof, wherein X is a group displaceable by a thiolate salt, and W and Y are as defined in claim 1;

(B) when, in the compound of general formula (I), Y represents a 5- or 6-membered unsaturated ring containing 1, 2, 3 or 4 nitrogen atoms, substituted by a pyrrol-1-yl group, the reaction with a 2,5-dialkoxytetrahydrofuran, of a corresponding compound wherein the said 5- or 6-membered unsaturated ring is instead substituted by an amino group;

(C) for the preparation of one enantiomer of a compound of formula I wherein p is 1:

(i) the replacement by hydrogen of a protective group Z in the corresponding enantiomer of a compound of the general formula IV

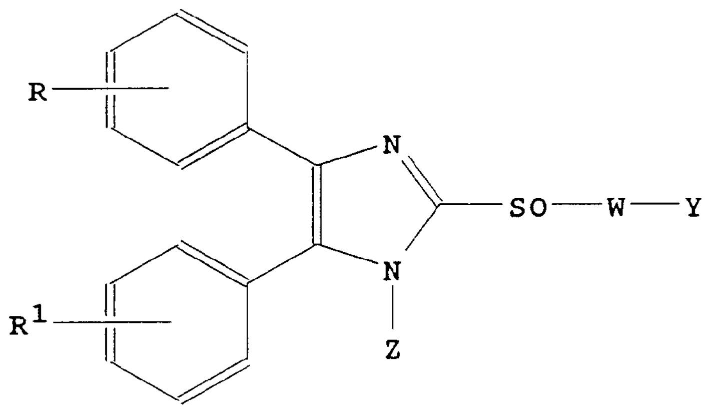

(IV)

wherein R, $R^1$, W and Y are as defined in claim 1, and Z represents a protective group;

(ii) the oxidation, using an enantio-selective oxidising system of a compound of general formula (I) wherein p is zero;

optionally followed by the conversion of a compound of general formula (I) into another compound of general formula (I);

optionally followed, when a mixture of enantiomers in which p is 1 is prepared, by the separation of enantiomers;

optionally followed by conversion of a compound of general formula (I) into a pharmaceutically acceptable salt thereof.

**10.** A pharmaceutical composition which comprises an imidazole derivative of formula (I) as defined in claim 1, or a pharmaceutically acceptable acid addition salt thereof, in association with a pharmaceutically acceptable carrier or coating.

**11.** A pharmaceutical composition useful in the treatment of a condition which can be ameliorated by administration of an inhibitor of acyl coenzyme-A: cholesterol-O-acyl transferase or of an inhibitor of the binding of thromboxane $TxA_2$ to its receptors which comprises an amount effective to ameliorate said condition of an imidazole derivative of general formula (I) as defined in claim 1 or a pharmaceutically acceptable acid addition salt thereof.

**12.** A method for the treatment of a human or animal host suffering from, or subject to, a condition which can be ameliorated by administration of an inhibitor of acyl coenzyme-A: cholesterol-O-acyl transferase or of an inhibitor of the binding of thromboxane $TxA_2$ to its receptors which comprises the administration to said host of an amount effective to ameliorate said condition of an imidazole derivative of general formula (I) as defined in claim 1, or a pharmaceutically acceptable acid addition salt thereof.

European Patent Office

# PARTIAL EUROPEAN SEARCH REPORT

which under Rule 45 of the European Patent Convention shall be considered, for the purposes of subsequent proceedings, as the European search report

Application Number
EP 92 30 6403

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | PATENT ABSTRACTS OF JAPAN, vol. 12, no. 40 (C-474)[2887], 5th February 1988; & JP-A-62 187 469 (TAISHO PHARMACEUT. CO., LTD) 15-08-1987<br>* Abstract *<br>--- | | C 07 D 403/14<br>A 61 K 31/415<br>C 07 D 403/12<br>C 07 D 401/14<br>A 61 K 31/40<br>A 61 K 31/44 |
| A | CHEMICAL ABSTRACTS, vol. 111, no. 11, 11th September 1989, page 746, column 2, abstract no. 97243c, Columbus, Ohio, US; & JP-A-01 40 467 (HISAMITSU PHARMACEUTICAL CO. INC.) 10-02-1989<br>* Abstract *<br>--- | | |
| P,A | WO-A-9 110 662 (RHONE-POULENC RORER S.A.)(25-07-1991)<br>----- | | |

TECHNICAL FIELDS SEARCHED (Int. Cl.5)

C 07 D
A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely : 4,6,8

Claims searched incompletely : 1-3,5,7,9-12

Claims not searched :

Reason for the limitation of the search:

See sheet -C-

| Place of search | Date of completion of the search | Examiner |
|---|---|---|
| THE HAGUE | 28-08-1992 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)

EP 92 30 6403 -C-

As the drafting of the claims is not clear and concise (Art.83-84 EPO) and encompasses such an enormous amount of products, a complete search is not possible on economic grounds [See Guidelines for Examination in the EPO Part B, Chapter III,2]. Guided by the spirit of the application and the inventive concept as disclosed in the descriptive part of the present application the search has been based on the examples.